# EUROPÄISCHE PATENTANMELDUNG

(11) **EP 2 722 016 A1**
(43) Veröffentlichungstag der Anmeldung: **23.04.2014**
(21) Anmeldenummer: 12189313.5
(22) Anmeldetag: 19.10.2012
(51) Int. Cl.: A61B 17/72

(54) **Aufsatz für das proximale Ende eines Marknagels**

(71) Anmelder: Rapido Med GmbH, 34560 Frtizlar (DE)
(72) Erfinder: Gregel, Siegbert, 34549 Edertal (DE)
(74) Vertreter: Fuchs

(57) **Zusammenfassung**

Die Erfindung befasst sich mit einem Aufsatz 24 für das proximale Ende eines Marknagels 1 mit einem Verbindungsbereich 25 an seinem ersten Ende 35 zum Verbinden des Aufsatzes 24 mit dem Marknagel 1 und mit einem Befestigungsbereich 26 zur Verbindung mit mindestens einer Befestigungsschraube zur Festlegung des Aufsatzes 24 in einem Knochen, wobei der Verbindungsbereich 25 ausgehend von seiner Abschlussfläche 27 eine sich in Richtung auf den Befestigungsbereich 26 erstreckende Aufnahmeausnehmung 28 aufweist, die zumindest in einem Teilbereich, ausgehend von der Abschlussfläche 27 eine sich verjüngende konische Verbindungsfläche 29 aufweist, wobei der Befestigungsbereich 26 hohl ausgebildet ist und in seiner Wand 30 mindestens ein Paar einander zugeordneter Durchbrechungen 31 ausgebildet ist, wobei die Durchbrechungen 31 so in der Wand 30 angeordnet sind, dass sie einen ersten Führungskanal 32 zur Aufnahme der Befestigungsschraube bilden, der durch die Mittellängsachse 33 des Aufsatzes 24 geht, wobei zwischen Befestigungsbereich 26 und Verbindungsbereich 25 eine Trennwand 47 ausgebildet ist, in der eine Durchgangsöffnung 34 entlang der Mittellängsachse 33 vorhanden ist, wobei am zweiten Ende 36 des Aufsatzes 24, das dem ersten Ende 35 abgewandt ist, eine Öffnung 37 vorhanden ist, die sich bis in den Befestigungsbereich 26 entlang der Mittellängsachse 33 erstreckt.

## Beschreibung

Die Erfindung befasst sich mit einem Aufsatz für das proximale Ende eines Marknagels sowie mit einem mit einem Marknagel verbundenen Aufsatz.

Es ist eine Vorrichtung zur Stabilisierung von Röhrenknochenbrüchen sowie von Gelenken bekannt aus DE 38 35 682 A1, die als Stabilisierungselemente ein- oder mehrgängig ineinander gewickelte Schraubenfedern aufweist, die zum Teil um eine Seele in Form eines elastisch verformbaren Drahtes oder eines Bündels von elastisch verformbaren Drähten herumgewickelt sind. Eine solche Schraubenfeder kann an den Enden mit einer Einrichtung zum Festlegen am Knochen ausgeführt sein. Nachteilig an dieser bekannten Vorrichtung ist die Unbestimmtheit der Lage der Schraubenfedern sowie die Unmöglichkeit einer Rotationsverhinderung wie auch einer Fixierung einzelner Bruchstücke im Bereich einer Fraktur, da sich die Federeigenschaften der Schraubenfedern dort nicht behindern lassen.

Weiterhin ist ein Nagelsystem für die intramedulläre Osteosynthese bei Schaftfrakturen aller Art bekannt, DE 197 31 298 A1, welches einen rohrförmigen Außenkörper mit Durchbrüchen aufweist, aus denen sich Haken nach außen erstrecken, die durch Betätigung eines Innenkörpers nach außen geschwenkt werden können, wodurch sich die Haken an der Innenwand eines Röhrenknochens anlegen können sollen. Dieses Nagelsystem ist extrem aufwändig und weist sehr viele Bauteile auf, kann aber prinzipbedingt nur Punktkontakte mit der Innenfläche eines Röhrenknochens erzeugen und diese auch nur in einem engen und über die Länge identischen Umfangsbereich.

Einfachere Marknägel zur Verriegelungsnagelung sind bekannt, deren Einbau während eines Eingriffs jedoch nur mittels intraoperativer Röntgendarstellung kontrolliert werden kann, was aufgrund der Dauer nicht nur für einen Patienten, sondern insbesondere für das OP-Personal äußerst belastend ist. Des Weiteren müssen solche einfachen Marknägel mittels Querschrauben im Knochen gesichert werden, wozu insbesondere bei langen Oberschenkelknochen, aber auch bei Oberarm- und Unterschenkelknochen aufwändige Zielgeräte erforderlich sind, um solche Querschrauben unter möglichst geringer Verletzung der Haut durch diese hindurch punktgenau setzen zu können. Diese zusätzlich in eine Extremität einzubringenden Operationswunden müssen nach einer Operation aber auch nach einer operativen Entfernung der Querschrauben aufwändig versorgt werden, was zusätzliche Belastungen für einen Patienten mit sich bringt.

Aus der EP 1 827 272 B1 ist ein intramedullärer Marknagel bekannt, der einfach aufgebaut ist, sehr praxisorientiert, leicht, schnell, sicher und bei nur geringem Einsatz von intraoperativer Röntgendarstellung eingesetzt werden kann, ohne dabei Eingriffe in unversehrte Bereiche einer Extremität zu erfordern. Dies wird dadurch erreicht, dass im vorderen Bereich des Marknagels ein Kopfstück angeordnet ist, an dem der Anfang eines Bandes festgelegt ist, welches um den Nagelkern herum aufgewickelt ist und das an dessen Ende im hinteren Bereich an einer Außenbuchse befestigt ist und welches bei einer Drehung entgegen seiner Aufwickelrichtung die radiale Kontur des Marknagels erweitert, wobei der Nagelkern an einer Innenbuchse befestigt ist, sodass die Innenbuchse innerhalb der Außenbuchse rotierbar und in Längsrichtung des Marknagels bewegbar ist. Mit diesem Marknagel kann allerdings kein axialer Druck auf den Frakturspalt bei Querbrüchen ausgeübt werden.

Aufgabe der Erfindung ist es deswegen, einen Aufsatz für das proximale Ende eines Marknagels zur Verfügung zu stellen, der eine einfache und sichere Verbindung mit einem implantierten Marknagel gewährleistet sowie eine entsprechende Verbindung aus einem Aufsatz und einem Marknagel aufzuzeigen.

Diese Aufgabe wird durch einen Marknagel mit den Merkmalen des Patentanspruchs 1 gelöst.

Durch die erfindungsgemäße konische Verbindungsfläche innerhalb des erfindungsgemäßen Aufsatzes wird im Zusammenspiel mit einem proximalen Ende eines Marknagels, der eine invers ausgebildete konische Klemmfläche aufweist, eine Konusklemmung erreicht, die zu variablen Winkelpositionen für in den Führungskanal, der durch die zwei Durchbrechungen in der Wand des Befestigungsbereichs gebildet wird, einzusetzende Befestigungsschrauben führt. Der Führungskanal kann entweder durch die Mittellängsachse des Aufsatzes verlaufen oder nicht zentral angeordnet sein. Durch eine Festlegeschraube, die in der Durchgangsöffnung in der Trennwand zwischen Verbindungs- und Befestigungsbereich angeordnet ist, kann eine leicht herzustellende und sichere Verbindung des Aufsatzes mit dem Marknagel erreicht werden, wenn die Festlegeschraube nach Durchtritt durch die Durchgangsöffnung in ein ebenfalls entlang der Mittellängsachse ausgebildetes Aufnahmegewinde, das dieselbe Größe wie die Durchgangsöffnung des Aufsatzes hat, am proximalen Ende des Marknagels eingreift. Die Durchgangsöffnung kann mit einer glatten Wand oder als Gewinde ausgebildet sein. Allerdings lassen sich zwei gleiche Gewinde nicht so gut zueinander befestigen. Die Festlegeschraube kann durch die Öffnung am zweiten Ende des Aufsatzes problemlos angezogen oder gelöst werden, da sich auch diese in derselben Achse - der Mittellängsachse des Aufsatzes - befindet, wie die Durchgangsöffnung.

Eine vorteilhafte Weiterbildung der Erfindung sieht vor, dass im Befestigungsbereich ein zweiter Führungskanal ausgebildet ist, dessen Durchbrechungen so ausgerichtet sind, dass er durch die Mittellängsachse verläuft, aber in Richtung der Mittellängsachse gesehen nicht deckungsgleich zum ersten Führungskanal ist. Dadurch kann eine bessere Verankerung des Aufsatzes im Knochen bzw. eine bessere Festlegung der Prothese erreicht werden.

Dies wird noch durch eine weitere vorteilhafte Weiterbildung der Erfindung verbessert, bei der im Befestigungsbereich ein dritter Führungskanal ausgebildet ist, dessen Durchbrechungen so ausgerichtet sind, dass er in Richtung der Mittellängsachse gesehen deckungsgleich zum ersten Führungsteil ist, jedoch entlang der Mittellängsachse beabstandet zum ersten Führungselement ist.

Eine weitere vorteilhafte Weiterbildung der Erfindung sieht vor, dass in der Öffnung am zweiten Ende ein Abschlussstück angeordnet ist, das entlang der Mittellängsachse beabstandet zum ersten Führungskanal ist. Dadurch kann nach erfolgter Festlegung des Aufsatzes am Marknagel eine glatte Abschlussfläche erreicht werden, die dazu führt, dass der Aufsatz bei Bedarf leicht wieder entfernt werden kann, da somit keine beziehungsweise nur eine sehr geringe Angriffsfläche für einwachsendes Gewebe angeboten wird.

Wie oben schon ausgeführt, wird durch die konische Klemmfläche im seinem Abschlussbereich, die im verbundenen Zustand spielfrei in der konischen Verbindungsfläche des Verbindungsbereichs angeordnet ist, eine hervorragende Verbindung zwischen Aufsatz und Marknagel erreicht. Dies wird noch dadurch verbessert, dass im Abschlussbereich des Marknagels entlang der Mittellängsachse ein Aufnahmegewinde ausgebildet ist, das dieselbe Größe wie die Durchgangsöffnung des Aufsatzes hat, und wobei der Verbindungsbereich des Aufsatzes mittels einer Festlegeschraube am Marknagel festgelegt ist, die durch die Durchgangsöffnung in das Aufnahmegewinde eingedreht ist.

Eine vorteilhafte Weiterbildung des mit dem Aufsatz verbundenen Marknagels sieht vor, dass im vorderen Bereich der Anfang eines Bandes - regelmäßig handelt es sich hierbei um ein äu βeres Band - festgelegt ist, welches um den Nagelkern herum aufgewickelt ist und dessen Ende im hinteren Bereich an einer Außenbuchse drehverriegelbar festgelegt ist, wobei sich bei einer Drehung entgegen seiner Aufwickelrichtung die radiale Kontur des Marknagels erweitert. Hierdurch wird erreicht, dass sich die Außenseite des Bandes an der Innenseite einer Durchgangsbohrung beziehungsweise unmittelbar an die Innenseite des Markraumes eines Röhrenknochens anlegen kann, beziehungsweise durch ein weiteres Drehen entgegen der Aufwickelrichtung eine radial wirkende Kraft erzeugt werden kann, mit der die Einzelteile eines Knochens optimal ausgerichtet und repositioniert geklemmt werden können, wodurch ein optimales Heilungsergebnis erzielt werden kann. Der technische Aufbau eines solchen Marknagels ist extrem einfach, leicht von jedem Notfallarzt zu verstehen und dadurch auch sehr sicher in der korrekten Anwendung. Auf den Einsatz von distalen Fixierschrauben des Nagels kann hier vollständig verzichtet werden, sodass eine postoperative Schnittwundenversorgung in diesem Bereich nicht mehr erforderlich ist. Durch die Klemmkraft des Bandes wird eine starke seitliche kortikale Abstützung und damit eine optimale Frakturstabilisierung erreicht, was des Weiteren zu einem schnelleren Ablauf der Frakturheilung führt. Frakturheilungsstörungen in Folge mangelhafter Frakturfixation können bei der Verwendung des Marknagels vollständig ausgeschlossen werden. Zur Entfernung des Marknagels wird das Band wieder stärker um den Nagelkern herum aufgewickelt, wodurch sich der Außendurchmesser des Marknagels wieder verringert, sodass er problemlos aus der Bohrung oder der Markhöhle herausgezogen werden kann. Da die Innenbuchse einen Außenkonus und die Außenbuchse einen zum Außenkonus koaxial ausgebildeten Innenkonus aufweisen, wobei Außenkonus und Innenkonus sich vorzugsweise jeweils in Richtung vom Kopfstück weg verjüngen, sich gegenüber liegen und zumindest abschnittsweise gleich ausgebildet sind, wobei die Flächen von Außenkonus und Innenkonus in einer Verriegelungsposition aneinander gepresst sind und in Entriegelungspositionen kein Kraft- und Formschluss zwischen Außenkonus und Innenkonus gegeben ist, wird eine ungewollte Lockerung der Fixation vermieden, da dieser die Pressung des Außenkonus in den Innenkonus bei einem Druck auf den Knochen in Längsrichtung des Marknagels noch verstärkt aber keine Bewegung wegen des Kraft- und Formschlusses mehr in diese Richtung möglich ist. Ferner kann durch die Verriegelung eine Kompression auf den Frakturspalt eines Röhrenknochens ausgeübt werden.

Eine vorteilhafte Weiterbildung der Erfindung sieht vor, dass die Innenbuchse am kopfstückfernen Ende des Außenkonus einen daran anschließenden Teil mit Außenzylinder und die Außenbuchse am kopfstückfernen Ende des Innenkonus einen daran anschließenden Teil mit Innenzylinder aufweisen, wobei Außenzylinder und Innenzylinder koaxial und im Wesentlichen spielfrei zueinander angeordnet sind. Dadurch wird eine gute und präzise Führung der Innenbuchse samt Nagelkern innerhalb der Außenbuchse trotz der teilweisen konischen Ausgestaltung erreicht, ohne dass ein Wackeln der beiden Teile zueinander möglich ist.

Eine weitere vorteilhafte Weiterbildung der Erfindung sieht vor, dass am kopfstückfernen Ende des Nagelkerns ein Endstück mit einem Außengewinde angeordnet ist, wobei zumindest ein Teil des Außengewindes nicht von der Außenbuchse in radialer Richtung überdeckt ist. Dadurch kann zum Abschluss des Marknagels eine Abschlusskappe aufgeschraubt werden, die bei einer entsprechenden Ausgestaltung der einzelnen Teile einen stufenlosen Übergang von der Außenbuchse zur Abschlusskappe ermöglicht. Bevorzugt ist dabei, dass das Endstück ein separates Teil zum Nagelkern ist und gegenüber diesem in seiner Lage fixiert ist.

Eine weitere vorteilhafte Weiterbildung der Erfindung sieht vor, dass der Verschiebeweg zwischen Entriegelungsposition und Verriegelungsposition höchstens 10 mm, bevorzugt höchstens 5 mm, besonders bevorzugt höchstens 3 mm, beträgt. Eine solche Bewegung der Au ßenbuchse auf das Kopfstück zu reicht aus, um eine Kompression zu erzeugen, durch die eine verbesserte Knochenheilung erzielt werden kann. Eine solche Kompression ist bei bestimmten Patientengruppen - wie Krebspatienten, Tumorpatienten, Drogensüchtigen, Alkoholikern und Patienten, die bestimmte Arzneimittel einnehmen - sehr nützlich.

Eine weitere vorteilhafte Weiterbildung der Erfindung sieht vor, dass an der Innenbuchse zwischen dem Außenkonus und dem Außenzylinder eine umlaufende Nut ausgebildet ist. Damit wird der aus fertigungstechnischen Notwendigkeiten in dem Bereich des Übergangs vom Konus zum Zylinder ausgebildete Grat entfernt.

Eine weitere vorteilhafte Weiterbildung der Erfindung sieht vor, dass am kopfstücknahen Ende des Innenkonus an der Außenbuchse eine erste Anschlagfläche und am kopfstücknahen Ende des Außenkonus an der Innenbuchse eine zweite Anschlagfläche ausgebildet sind, die beide im Wesentlichen senkrecht zur Längsrichtung des Marknagels orientiert sind. Dadurch ist die Innenbuchse mit Nagelkern unverlierbar mit der Außenbuchse verbunden und die beiden Teile können vor und während des Einsetzens des Marknagels nicht zu weit ihre relative Position zueinander verändern.

Eine weitere vorteilhafte Weiterbildung der Erfindung sieht vor, dass die erste Anschlagfläche Teil eines von der Außenbuchse separaten Führungsteils ist, wobei das Führungsteil fest mit der Außenbuchse verbunden ist. Dadurch kann bei der Herstellung des Marknagels durch einfache Art und Weise die nötige Montage von Innenbuchse zu Außenbuchse an einer günstigen Stelle vorgenommen werden. Aufgrund der Ausrichtung der beiden Konen ist es nicht möglich, die Innenbuchse von der kopfstückfernen Seite in die Außenbuchse einzuführen. Die feste Verbindung des Führungsteils mit der Außenbuchse erfolgt bevorzugt durch Verschweißen, ist aber auch durch alle anderen, dem Fachmann bekannten Verbindungsmethoden möglich.

Eine weitere vorteilhafte Weiterbildung der Erfindung sieht vor, dass der Nagelkern gerade ausgebildet und mit der Innenbuchse fest verbunden ist. Hierbei handelt es sich um eine einfache und sichere Möglichkeit, diese beiden Teile miteinander zu verbinden. Der Nagelkern muss dabei gerade ausgebildet sein, da das Kopfstück ansonsten bei der Drehung der Innenbuchse samt Nagelkern eine Kreisbewegung vollführen würde, was in einem Knochen nicht möglich ist.

Eine weitere vorteilhafte Weiterbildung der Erfindung sieht vor, dass die Innenbuchse und/oder der Nagelkern hohl ausgebildet sind. Dadurch wird Masse und somit Gewicht eingespart, was aufgrund des reduzierten Materialbedarfs zu einer Preisreduzierung führt und trotzdem eine ausreichende Stabilität des Marknagels sicherstellt.

Eine weitere vorteilhafte Weiterbildung der Erfindung sieht vor, dass die Innenfläche des Führungsteils der Außenbuchse zylindrisch ist und die darin angeordneten Teile der Außenfläche der Innenbuchse oder des Nagelkerns im Wesentlichen dazu spielfrei ausgebildet sind. Dadurch wird zusätzlich oder alternativ zu der Ausgestaltung mit den oben schon angegebenen Innen- und Außenzylinder am kopfstückfernen Ende der Außen- und der Innenbuchse eine gute und präzise Führung der Innenbuchse samt Nagelkern innerhalb der Außenbuchse trotz der teilweisen konischen Ausgestaltung erreicht, ohne dass ein Wackeln der Teile zueinander möglich ist.

Als besonders vorteilhaft ist eine Ausführung hervorzuheben, bei der im vorderen Bereich des Marknagels der Anfang eines weiteren Bandes - regelmäßig eines inneren Bandes - festgelegt ist, welches unter dem ersten Band - regelmäßig einem äußeren Band - um den Nagelkern herum aufgewickelt ist und dessen Ende im hinteren Bereich des Marknagels drehverriegelbar um die Innenbuchse festgelegt ist. Ein solches (inneres) Band vergrößert bei einer Drehung entgegen seiner Aufwicklungsrichtung ebenfalls den Außendurchmesser des Marknagels beziehungsweise legt sich mit seiner Außenseite an der Innenseite des ersten (äußeren) Bandes an und stabilisiert so das Band und damit den gesamten Marknagel. Diese Konstruktion ermöglicht die Verwirklichung einer hohen Steifigkeit, die etwa der eines dünnen Rohres entsprechen kann.

Gemäß weiterer vorteilhafter Ausführungsformen der Erfindung können auch mehrere innere oder äußere Bänder gleich- oder gegensinnig sowie ein- oder mehrgängig um den Nagelkern herum aufgewickelt sein, wobei bei einer einlagigen Version der inneren Bänder diese unmittelbar um den Nagelkern herum aufgewickelt sind und bei einer einlagigen Ausführungsform der äußeren Bänder diese um die inneren Bänder herum aufgewickelt sind. Dieser Variationsreichtum erlaubt es, für die häufigsten Arten von Frakturen von Knochen beliebiger Größe jeweils einen optimal angepassten Marknagel zur Verfügung stellen zu können, da die geforderte Festigkeit quasi beliebig hoch eingestellt werden kann.

Besonders vorteilhaft ist es, wenn die radialen Außenflächen des inneren Bandes und die radialen Innenflächen des äußeren Bandes als gegenseitige Funktionsflächen rau ausgebildet sind, sodass die gemeinsamen Kontaktflächen der Bänder Stabilisierungspunkte eines in eine Durchgangsbohrung eines Knochens eingesetzten und verspannten Marknagels in Form einer kraft- und reibschlüssigen Verbindung erzeugen. Hierdurch wird die Stabilität des Marknagels weiter vergrößert.

Bei einer weiteren vorteilhaften Ausgestaltung des Gegenstandes der Erfindung ist der Nagelkern über seine gesamte Länge hohl ausgebildet und von einer Hülse und/oder einer Welle durchtreten, sodass am vorderen Bereich des Kopfstückes des Marknagels der Anfang eines inneren und/oder äußeren Bandes festgelegt werden kann, wobei vom hinteren Ende des Marknagels dieses über einen entsprechend ausgebildeten Verriegelungsmechanismus von hinten gegen seine Drehrichtung abgewickelt und aufgespannt werden kann. Bei einer praxisorientierten Ausführungsform kann dabei eine deutlich höhere Anzahl von Windungen und hier nur ein einzelnes, von der als Kopfstück ausgebildeten Spitze aus gegensinnig aufgewickeltes Band, dessen innere Wicklungen das innere Band bilden und die äußeren Wicklungen das äußere Band, verwendet werden. Der gegenständliche Nagelkern ist hier bis auf einen Teil im Verriegelungsmechanismus vollständig reduziert, beziehungsweise wird durch einen freien Luftraum ersetzt. Eine solche Version ermöglicht ein nach außen kräftefreies Verspannen des Marknagels, wie bei allen anderen Versionen auch, ohne dass mechanische Rückstellkräfte von einem Knochen aufgenommen oder in diesen eingeleitet werden müssten.

Eine vorteilhafte Weiterbildung des Marknagels besitzt etwa zwei äußere Bänder von denen eines vom vorderen Bereich des Marknagels aus und eines vom hinteren Bereich des Marknagels aus aufdrehbar sind, sodass eine optimale Anpassung des jeweiligen Bandes an den sich von einer Markraumtaille beidseitig trompetenhaft erweiternden Markraum und damit eine optimale seitliche kortikale Abstützung möglich wird.

Der Marknagel kann je nach Bedarf gerade oder gebogen ausgebildet oder auch entsprechend der geforderten Raumform plastisch verbiegbar sein.

Weiterhin kann der Nagelkern eines Marknagels in vorteilhafter Weise mehrteilig ausgebildet sein, sodass sich diese Teile gegeneinander verdrehen lassen. Dies erweitert die Möglichkeiten zur konstruktiven Gestaltung sehr wesentlich.

Bei einer weiteren sehr vorteilhaften Ausführungsform der Erfindung sind zwei gegeneinander aufgewickelte Bänder an ihren vorderen Enden miteinander verbunden oder aber ein Band von vorne herein einteilig ausgebildet und von seinem vorderen Ende aus gegensinnig um den Nagelkern aufgewickelt, sodass der Nagelkern verkürzt ausgebildet sein kann und sich nur bis zu einem Teilbereich in Richtung des vorderen Endes des Marknagels erstreckt. Der Marknagel kann bei einer solchen Ausgestaltung dünner sein als bei durchgehendem Nagelkern. Bei einer extremen Ausführungsform dieser Ausgestaltung ist der Nagelkern sogar bis auf den Verriegelungsmechanismus reduziert, wodurch quasi im Einbauzustand ein stabiles starres Netzgitter als Marknagel übrig bleibt, was zu einer enormen Materialeinsparung führt. Eine solche Ausführungsform ist besonders bei kleinen Röhrenknochen - wie beispielsweise Ulna und Radius - geeignet.

Eine weitere vorteilhafte Weiterbildung der Erfindung sieht vor, dass der Marknagel aus Stahl besteht. Aufgrund der geringeren Biokompatibilität von Stahl gegenüber Titan wird das Einwachsen auf ein Minimum reduziert. Stahl ist nicht thrombogen, toxisch, allergen, fibrogen oder karzinogen. Es erfolgt kein Abbau zellulärer Elemente, keine Veränderung an Plasmaproteinen und Enzymen und auch keine Gewebsnekrose. Stahl ist korrosionsfest, da keine Ionisierung metallischer Atome erfolgt, degradationsfest und auslaugungsfest bei Kontakt mit Blut oder Körperflüssigkeit. Es erfolgt kein Einwachsen osteoblastenähnlicher Knochenzellen durch die mechanische Strukturierung der Implantatoberfläche und die physikalisch-chemischen Eigenschaften des Oberflächenwerkstoffs können eingestellt werden, sodass eine Einkapselung des Implantats anstatt eines Einwachsens erfolgt und eine einfache Metallentfernung gewährleistet ist.

Erfindungsgemäß ist auch die Verwendung des hierin beschriebenen Marknagels zur Ausübung eines Kompressionsdrucks auf einen Frakturspalt in einem Röhrenknochen.

Eine vorteilhafte Ausgestaltungen der Erfindung sowie weitere Vorteile werden darüber hinaus anhand der Zeichnung erläutert. Im Einzelnen zeigen:
- Figur 1: eine teilgeschnittene Ansicht eines Marknagels mit Abschlusskappe,
- Figur 2: eine geschnittene Teilansicht des Marknagels, insbesondere eines Verriegelungsmechanismus im entriegelten Zustand,
- Figur 3: einen Schnitt gemäß Figur 2 mit dem Verriegelungsmechanismus im verriegelten Zustand,
- Figur 4: einen Längsschnitt entlang der Mittellängsachse und in der Ebene IV-IV in Figur 6 durch einen erfindungsgemäßen Aufsatz,
- Figur 5: einen Längsschnitt wie in Figur 4 aber in der Ebene V-V in Figur 6,
- Figur 6: einen Querschnitt durch den Aufsatz der Figuren 4 und 5 in einer dazu senkrechten Ebene in seinem Befestigungsbereich,
- Figur 7: eine Seitenansicht eines Abschlussstücks,
- Figur 8: eine Draufsicht auf das Abschlussstück von rechts in der Figur 7 und
- Figur 9: einen Teilausschnitt im Längsschnitt eines Aufsatzes, der mit einem Marknagel verbunden ist.

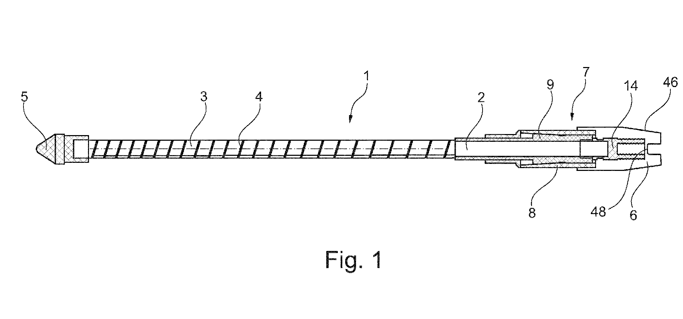

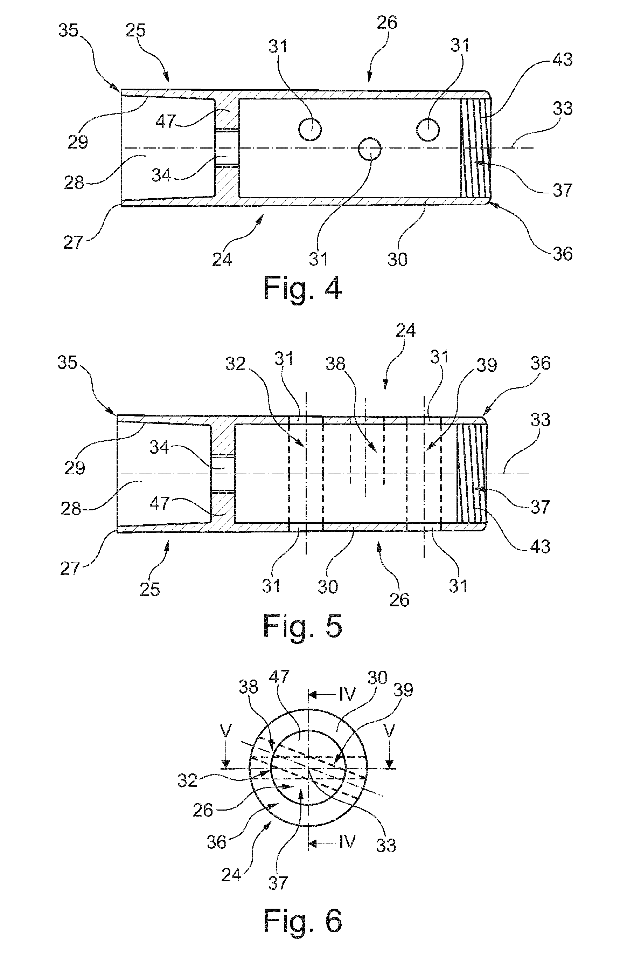

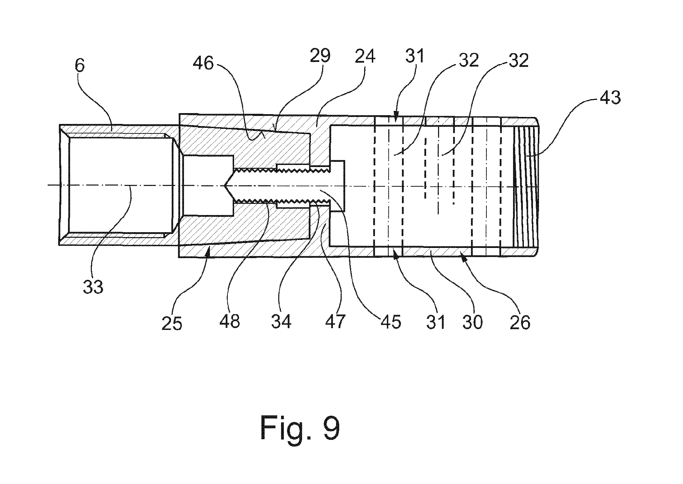

Zuerst wird anhand der Figuren 1 bis 3 ein Marknagel 1 beschrieben, der mit einem erfindungsgemäßen Aufsatz 24 verbunden werden kann, um die Vorteile des Aufsatzes 24 ausnutzen zu können. Im Anschluss daran wird anhand der Figuren 4 bis 8 ein Ausführungsbeispiel eines erfindungsgemäßen Aufsatzes 24 beschrieben. Anhand von Figur 9 wird die Verbindung zwischen Marknagel 1 und Aufsatz 24 beschrieben.

Der erfindungsgemäße Aufsatz 24 ist als eine Art Modul auf unterschiedliche Arten von Marknägeln 1 abstimmbar. Es kann beispielsweise im Bereich der Metaphyse eingesetzt werden, aber genauso auch im Bereich der Epiphyse. Man könnte somit auch von einem Metaphysemodul oder einem Epiphysemodul sprechen. Bei einem Epiphysemodul kann auch anstatt den Aufsatz 24 mit Befestigungsschrauben im Knochen zu verankern, ein Gelenkimplantat über die Befestigungsschraube mit dem Aufsatz 24 verbunden werden.

Der Marknagel 1, wie er in den Figuren 1 bis 2 dargestellt ist, besteht aus einem Nagelkern 2, um den von seinem Kopfstück 5 ein inneres Band 4 und ein äußeres Band 3 gegensinnig zueinander aufgewickelt sind, wobei die vorderen Enden der Bänder 3, 4 etwa über ein Laser-Schweißverfahren am Kopfstück 5 ortsfest angeordnet sind. Der Nagelkern 2 weist an seinem kopfstücknahen Ende einen geringeren Durchmesser auf und ist dort in das Kopfstück 5 eingeschoben und mit diesem fest verbunden - beispielsweise durch Verschweißen. Im hinteren Bereich des Marknagels 1 sind die Enden der Bänder 3, 4 an einer Außenbuchse 8 beziehungsweise einer Innenbuchse 9 des Verriegelungsmechanismus 7 angeordnet und dort beispielsweise ebenfalls mittels eines Lasers verschweißt. Die gegensinnig aufgewickelten Bänder 3, 4 überdecken sich an Kontaktflächen, wobei die zueinander gerichteten Oberflächen der Bänder 3, 4 so ausgebildet sind, dass dort eine reibschlüssige Fixierung erfolgt, die den gesamten Marknagel 1 stabilisiert. Dadurch ergibt sich eine verbesserte Fixierung einer Fraktur.

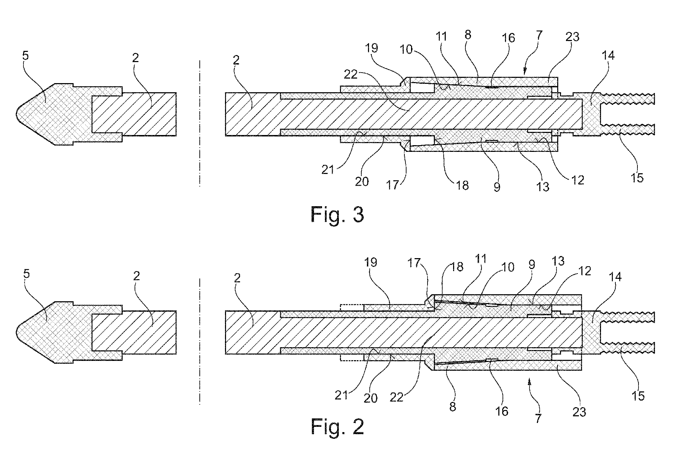

Die in Figur 1 beispielhaft dargestellte Ausführungsform des äußeren Bandes 3 weist Windungen auf, die im Bereich des Kopfstücks 5 eine geringere Breite aufweisen als im Bereich der Außenbuchse 8. Zur dadurch erzielten Wirkung folgen unten weitergehende Ausführungen. Ein Endstück 14 ist mit dem kopfstückfernen Ende des Nagelkerns 2 fest verbunden, beispielsweise durch Verschweißen. Auf dieses wird im verriegelten Zustand, der in Figur 1 dargestellt ist und in Figur 3 noch näher erläutert wird, eine Abschlusskappe 6 geschraubt, deren Funktion weiter unten noch näher erläutert wird. Die Abschlusskappe 6 weist im Bereich seines in Figur 1 rechts dargestelltes Endes eine konische Klemmfläche 46 auf. Der Konuswinkel ist - wie auch in Figur 9 - aus Gründen der besseren Erkennbarkeit übertrieben groß dargestellt. Seine wirkliche Größe liegt bei 1,43°, wie dies in den Figuren 4 und 5 dargestellt ist.

In den Figuren 2 und 3 sind Längsschnitte durch den Marknagel 1 der Figur 1 dargestellt, wobei die Bänder 3, 4 aus Gründen der Übersichtlichkeit nicht dargestellt sind.

Die erfindungsgemäße Neuerung des Marknagels 1 ist in seinem Verriegelungsmechanismus 7 zu sehen.

Die mit dem äußeren Band 3 (siehe Figur 1) verbundene Außenbuchse 8 ist zweiteilig ausgebildet. Über eine Schweißnaht 22 - andere Verbindungsmethoden sind ebenfalls möglich - ist ein Führungsteil 19 (in den Figuren 2 und 3 ist dies der linke Teil der Außenbuchse 8) mit einem Hauptteil 23 verbunden. Der Hauptteil 23 weist ausgehend von der Schweißnaht 22 nach rechts eine inneren Oberfläche in der Form eines Innenkonus 11 auf, der sich nach rechts verjüngt. Direkt im Anschluss an diesen Innenkonus 11 schließt sich einstückig (die beiden Bereiche können auch als separate, fest miteinander verbundene Teile ausgeführt sein) ein Bereich an, dessen Oberfläche die Form eines Innenzylinders 13 aufweist. Der Durchmesser des Innenzylinders 13 ist gleich groß wie der (kleinste) Durchmesser des Innenkonus 11 an seinem rechten Ende. Innenzylinder 13 und Innenkonus 11 sind koaxial ausgerichtet entlang der Mittelachse des Nagelkerns 2.

Der Führungsteil 19 ist in seinem schweißnahtfernen Bereich als Zylinder ausgebildet, der koaxial zum Innenzylinder 13 und Innenkonus 11 angeordnet ist, dessen Innenfläche 20 einen Durchmesser hat, der geringer ist als der an die Schweißnaht anschließende (größte) Durchmesser des Innenkonus 11. Im Bereich der Schweißnaht 22 weist der Führungsteil 19 deshalb eine Schulter auf, die senkrecht auf der Längsachse des Nagelkerns 2 steht und über die er mit dem Hauptteil 23 verschweißt ist. Dadurch wird im Bereich der Schulter eine erste Anschlagfläche 17 gebildet, die sich senkrecht zur Längsachse des Nagelkerns 2 erstreckt.

Die Innenbuchse 7 ist verschiebbar entlang der Längsachse des Nagelkerns 2 innerhalb der Außenbuchse 8 angeordnet. Sie ist zwar zum Nagelkern 2 um dessen Längsachse - einem Gleitlager vergleichbar - rotierbar, aber nicht entlang dieser Achse zu ihm verschiebbar. Dies wird dadurch erreicht, dass der Durchmesser des Nagelkerns 2 am kopfstückfernen Ende geringer ist als in seinem Mittelabschnitt, wodurch ein Anschlag gebildet wird, über den der Nagelkern 2 am kopfstücknahen Ende der Innenbuchse 9 anschlägt; gleichzeitig wird am kopfstückfernen Ende der Innenbuchse 9 durch das fest mit dem Nagelkern 2 verbundenen Endstück 14 ein weiterer Anschlag für den Nagelkern 2 gegenüber der Innenbuchse 9 ausgebildet.

Sie weist im Wesentlichen drei Abschnitte auf, die für ihre Funktionalität im Zusammenspiel mit der Außenbuchse 8 von Bedeutung sind: im linken, kopfstücknahen Bereich eine zylindrische Außenfläche 21, der daran anschließende Bereich weist die Form eines Außenkonus 10 auf, an den sich zum kopfstückfernen Ende der Innenbuchse 9 hin ein Bereich in der Form eines Außenzylinders 12 anschließt.

Die zylindrische Außenfläche 21 ist so dimensioniert, dass sie gerade spielfrei in der zylindrischen Innenfläche 20 des Führungsteils 19 der Außenbuchse 8 läuft.

Der Außenzylinder 12 ist so dimensioniert, dass er gerade spielfrei im Innenzylinder des Hauptteils 23 der Au βenbuchse 8 läuft.

Der dazwischenliegende Bereich des Außenkonus 10 ist in Richtung der Längsachse des Nagelkerns 2 kürzer ausgebildet als der Innenkonus 11 der Außenbuchse 8. Im Übergangsbereich zwischen Außenkonus 10 und Außenzylinder 12 ist eine umlaufende Nut 16 ausgebildet. Am Übergang des Außenkonus 10 in die Nut 16 ist der Durchmesser des Außenkonus (sein kleinster) geringfügig größer als derjenige des Innenkonus 11 der Außenbuchse 8 an seinem Übergang zum Innenzylinder 13. Durch die Ausführung der Nut 16 in diesem Übergangsbereich wird der aus fertigungstechnischen Notwendigkeiten dort ausgebildete Grat entfernt. Der Öffnungswinkel des Außenkonus 10 ist gleich wie derjenige des Innenkonus 11 der Außenbuchse 8. Im Bereich des Übergangs von der Außenfläche 21 zum Außenkonus 10 weist die Innenbuchse 9 eine senkrecht zur Längsachse des Nagelkerns 2 stehende zweite Anschlagfläche 18 auf, die kleiner ist als die erste Anschlagfläche 17 am kopfstückfernen Ende des Führungsteils 19 der Au ßenbuchse 8.

Am kopfstückfernen Ende der Innenbuchse 9 ist der Nagelkern 2 mit einem Endstück 14 mittels einer geeigneten, dem Fachmann bekannten Verbindungsmethode fest miteinander verbunden. Das Endstück 14 weist an seinem kopfstückfernen Ende ein Au ßengewinde 15 auf.

Außenzylinder 12, Außenkonus 10 und Außenfläche 21 der Innenbuchse 9 sind koaxial zueinander und auch koaxial zu den entsprechenden Teilen der Außenbuchse 8 angeordnet. Somit kann sich die Innenbuchse 9 über eine gewisse Länge innerhalb der Außenbuchse 8 bewegen. Die beiden Extrempunkte dieser Bewegungsmöglichkeit sind in den Figuren 2 und 3 dargestellt.

Figur 2 stellt den Marknagel 1 in seinem entriegelten Zustand dar, wie er vor und während des Einführens in den Knochen vorliegt. Hierbei ist die Innenbuchse 9 so in der Außenbuchse 8 angeordnet, dass ihre zweite Anschlagfläche 18 an der ersten Anschlagfläche 17 der Außenbuchse 8 anschlägt. Hierbei berührt der Außenkonus 10 der Innenbuchse 9 den Innenkonus 11 der Außenbuchse 8 nicht. Die Innenbuchse 9 kann somit frei - abgesehen von den Bändern 3, 4, die dies erschweren - innerhalb der Außenbuchse 8 um die Längsachse des Nagelkerns 2 rotiert werden.

Nach dem Einführen des Marknagels 1 in den Knochen und Erreichen der gewünschten Position wird zur Fixierung des Marknagels 1 ein Aufdrehen durchgeführt. Hierzu wird die Innenbuchse 9 in der Außenbuchse 8 um die Längsachse des Nagelkerns 2 rotiert, sodass eine Aufweitung der Bänder 3, 4 erfolgt.

Die Bänder 3, 4 (siehe Figur 1) bestehen bevorzugt aus federelastischem Material, etwa aus chirurgischem Stahl, Titan oder körperfreundlichem Kunststoff. Dadurch lassen sich die gewünschten Eigenschaften des Marknagels 1 hinsichtlich der erforderlichen Kontaktkraft wie auch die Art und der Ablauf der Verformung, vorteilhafterweise zunächst von dem vorderen Bereich des Marknagels 1 über die Markraumtaille bis zum sich wieder erweiternden Ende eines Markraumes optimal festlegen. Um eine Aufweitung zu erreichen, bei der sich das äußere Band 3 an die Innenkonturen des Markraumes eines Röhrenknochens anlegt, sind das innere Band 4 und/oder das äußere Band 3 in unterschiedlichen Bereichen des Nagelkerns 2 unterschiedlich breit oder dick ausgebildet (siehe Figur 1) oder aber das innere Band 4 und/oder das äußere Band 3 sind in unterschiedlichen Bereichen des Nagelkerns 2 unterschiedlich stark elastisch vorgespannt. Denkbar ist es auch, dass beispielsweise das äußere Band 3 über seine gesamte Länge oder zumindest teilweise, vom Kopfstück 5 aus gesehen etwa über die ersten 2/3 seiner Länge einen sich kontinuierlich verändernden Querschnitt, insbesondere eine zunehmende Breite aufweist (siehe Figur 1), um die geforderte Funktion zu erfüllen. Eine solche vorteilhafte Konstruktion ermöglicht es auch, bei nicht vorgespannten Bändern 3, 4, dass sich das äußere Band 3 beim Aufdrehen gegen den Drehsinn zunächst im Bereich des Kopfstückes 5 des Marknagels 1 an die Innenkontur eines Knochens anlegt und sich erst beim weiteren Aufdrehen zunächst im mittleren Bereich der Markraumtaille eines Röhrenknochens und anschließend im Endbereich des Marknagels 1 an den sich wieder erweiternden Bereich des Markraumes anlegt.

Ist die gewünschte Aufdrehung der Bänder 3, 4 erreicht, erfolgt eine Überführung des Marknagels 1 in seinen verriegelten Zustand, wie er in Figur 3 dargestellt ist. Hierzu wird die Außenhülse 8 mit einem geeigneten Montagewerkzeug in Richtung auf das Kopfstück 5 zu bewegt, bis ein Klemmsitz des Außenkonus 10 der Innenbuchse 9 am Innenkonus 11 der Außenbuchse 8 gegeben ist. Dies wird schon nach einem Verschiebeweg von 3 mm erreicht. In diesem verriegelten Zustand wird eine weitere Rotation der Innenbuchse 9 in der Außenbuchse 8 ebenso wie eine axiale Verschiebung der Teile zueinander unterbunden. Der Marknagel 1 ist unverrückbar im Knochen fixiert.

Um eine ungewollte Lösung des Klemmsitzes, beispielsweise bei Erschütterungen, zu unterbinden, wird nach Erreichen der in Figur 3 gezeigten Position auf das Au ßengewinde 15 des Endstücks 14 eine mit einem geeigneten Gewinde versehene Abschlusskappe 6 (siehe Figur 1) als Sicherungsmutter aufgeschraubt, bis diese mit ihrem linken Rand an dem rechten, kopfstückfernen Rand des Hauptteils 23 der Außenbuchse 8 anschlägt. Dann ist gewährleistet, dass auch bei Erschütterungen keine Lageveränderung der Innenbuchse 9 gegenüber der Außenbuchse 8 erfolgen kann. Die Abschlusskappe 6 verjüngt sich auf ihr freies Ende hin konisch und bildet eine Klemmfläche 46. Der Konuswinkel der Klemmfläche 46 ist gleich groß wie derjenige einer konischen Verbindungsfläche 29 an einem erfindungsgemäßen Aufsatz 24 (siehe Figuren 4, 5 und 9) -jeweils gegenüber der Mittellängsachse 33 gemessen.

Damit wird ein Marknagel 1 zur Verfügung gestellt, der trotz geringer Anzahl an benötigten Einzelteilen einen Verriegelungsmechanismus 7 aufweist, der auch bei axialem Druck seine vom Operateur eingestellte Spannung und Position nicht ändert.

In den Figuren 4 bis 6 ist eine Ausführungsform eines erfindungsgemäßen Aufsatzes 24 aus unterschiedlichen Blickrichtungen - Figuren 5 und 6 in Längsschnitten - dargestellt.

Die äußere Form des Aufsatzes 24 ist im Wesentlichen zylindrisch; er ist bis auf - unten noch näher erläuterte - Durchbrechungen 31 in seiner Wand 30 symmetrisch zu seiner Mittellängsachse 33 aufgebaut. Da die Darstellungen der Figuren 4 und 5 senkrecht zueinander verlaufende Längsschnitte durch den Aufsatz 24 jeweils durch die Mittellängsachse (zur Ausrichtung der Längsschnitte siehe Figur 6) zeigen, werden diese im Folgenden zusammen beschrieben.

Der Aufsatz 24 weist einen Verbindungsbereich 25 auf, über den der Aufsatz 24 mit dem Marknagel 1 verbunden wird. Hierzu ist in seiner in den Figuren 4 und 5 links angeordneten Abschlussfläche 27 seines ersten Endes 35 eine Aufnahmeausnehmung 28 ausgebildet, die sich ausgehend von der Abschlussfläche 27 in Richtung des zweiten Endes hin konisch verjüngt. Hierdurch wird eine konische Verbindungsfläche 29 gebildet, deren Konuswinkel - die Neigung der Oberfläche der Verbindungsfläche gegenüber der Mittellängsachse 33 - gleich groß ist wie der Konuswinkel der konischen Klemmfläche 46 an der Abschlusskappe 6 des Marknagels 1 (siehe Figuren 1 und 9).

Die konische Verbindungsfläche 29 endet an einer Trennwand 47, die den Verbindungsbereich 25 von einem Befestigungsbereich 26 trennt. In der Trennnwand 47 ist eine Durchgangsöffnung 34 um die Mittellängsachse 33 ausgebildet. Die Größe der Durchgangsöffnung 34 hängt von der Größe des Aufsatzes 24 ab und ist auf die Gewindegröße des Aufnahmegewindes 48 der Abschlusskappe 6 abgestimmt, damit die Festlegeschraube 45 in die beiden Gewinde eingeschraubt werden kann und somit eine feste Verbindung zwischen Marknagel 1 und Aufsatz 24 erreicht werden kann. Regelmäßig handelt es sich um ein M4- oder M5-Gewinde. Der Durchmesser der Aufnahmeausnehmung 28 an der Trennwand ist gleich groß wie der Durchmesser der konischen Klemmfläche 46 am freien Ende der Abschlusskappe 6. Dies führt zu einem sehr guten Klemmsitz zwischen Abschlusskappe 6 und Aufsatz 24, wenn diese miteinander verbunden sind, wie es in Figur 9 dargestellt ist. Der Konuswinkel beträgt im Ausführungsbeispiel nur 1,43°, wobei dieser in den Figuren 1, 4, 5 und 9 zur besseren Erkennbarkeit größer dargestellt ist.

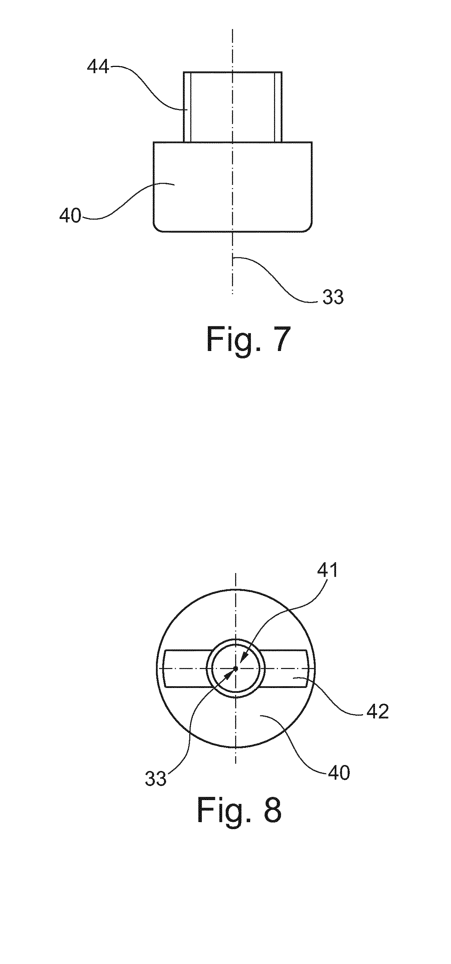

An die Trennwand 47 schließt sich in Richtung auf das zweite Ende 36 der Befestigungsbereich 26 an. Dieser wird durch die zylindrische Wand 30 begrenzt und ist innen hohl. An seinem zweiten Ende weist der Aufsatz 24 eine Öffnung 37 auf, in der ein Verbindungsbereichinnengewinde 43 ausgebildet ist. Die Gewindegröße hängt von der Größe der Öffnung 37 ab und ist auf ein Abschlussstückaußengewinde 44 eines Abschlussstücks 40 (siehe Figuren 7 und 8) abgestimmt. Regelmäßig handelt es sich um ein M8- oder M10-Gewinde. Diese Gewinde können auch als Linksgewinde ausgeführt sein. Anstatt dieser Gewinde ist auch eine Ausbildung als Feingewinde möglich. Mit einem Feingewinde wird Platz eingespart, sodass noch ein weiterer Führungskanal gesetzt werden kann. Mit einem solchen weiteren Führungskanal kann man weiter nach proximal kommen, um mit einer Befestigungsschraube ein sehr weit sitzendes proximales Fragment zu fassen.

In der Wand 30 sind drei Paare von Durchbrechungen 31 ausgebildet, von denen jeweils zwei gegenüberliegende einen Führungskanal 32, 38, 39 für jeweils eine Befestigungsschraube, die zur Befestigung im Knochen dienen, bilden. Im Ausführungsbeispiel sind drei solche Führungskanäle 32, 38, 39 dargestellt. Die Durchbrechungen 31 sind so angeordnet, dass jeder Führungskanal 32, 38, 39 durch die Mittellängsachse 33 verläuft und senkrecht auf dieser steht. Der erste Führungskanal 32, der am nächsten zur Trennwand 47 angeordnet ist, und der dritte Führungskanal, der am nächsten zur Öffnung 37 angeordnet ist, sind in Richtung der Mittellängsachse 33 gesehen deckungsgleich (und nur entlang der Mittellängsachse beabstandet zueinander angeordnet). Der zweite Führungskanal 38 ist zwischen dem ersten Führungskanal 32 und dem dritten Führungskanal 39 angeordnet und um einen Winkel im Bereich von 20° um die Mittellängsachse 33 gegenüber den anderen beiden Führungskanälen 32, 39 gedreht. Dadurch können mehrere Befestigungschrauben verwendet werden, die mehrere Knochenfragmente unter unterschiedlichen Winkeln zueinander fixieren können, was die Fragmenterfassung flexibler macht.

Die Ausrichtung der Führungskanäle 32, 38, 39 ist gut aus Figur 6 zu ersehen, die eine Blickrichtung entlang der Mittellängsachse 33 mit Blick auf das zweite Ende 36 mit darin ausgebildeter Öffnung 37 zeigt. Die Führungskanäle 32, 38, 39 sind dabei strichliert dargestellt, da sie nicht körperlich vorhanden sind, da der Befestigungsbereich 26 innerhalb der Wand 30 hohl ausgebildet ist.

In den Figuren 7 und 8 ist ein Abschlussstück 40 dargestellt, das mit seinem Abschlussstückaußengewinde 44 in die Öffnung 37 des Aufsatzes 24 eingeschraubt wird, nachdem der Aufsatz 24 mit dem Marknagel 1 fest verbunden wurde und die Befestigungsschrauben gesetzt wurden. Damit wird ein guter Abschluss des Aufsatzes 24 erreicht, der keine Öffnungen und Kanten aufweist.

Durch die Konusklemmung zwischen Verbindungsfläche 29 am Aufsatz 24 und Klemmfläche 46 an der Abschlusskappe 6 des Marknagels 1 wird eine variable Winkelposition der Befestigungsschrauben erreicht. Das Abschlussstück 40 weist an seinem Kopf einen Schlitz 42 auf, der dazu dient, das Abschlussstück 40 (siehe Figuren 7 und 8) mit einem Instrument zu entfernen. Mit dem Instrument wird auch die Abschlusskappe 6 des Marknagels 1 konnektiert oder diskonnektiert. Da das Instrument ohnehin steril auf dem OP-Tisch aufliegen muss, bietet es sich an, dieses auch für die Abschlusskappe 6 und das Abschlussstück 40 gleichermaßen zu nutzen, damit nicht unnötigerweise weitere Instrumente zur Verfügung gestellt werden müssen. Um die Mittellängsachse 33 herum ist eine Gewindedurchbrechung 41 vorhanden, durch die ein Zielgerät an der Kombination aus Marknagel 1 und Aufsatz 24 festgelegt werden kann, damit die Befestigungsschrauben problemlos so durch den Knochen getrieben werden können, dass die von den Durchbrechungen 31 festgelegten Führungskanäle 32, 38, 39 problemlos getroffen werden können.

Die Länge des Aufsatzes 24 vom ersten Ende 35 bis zum zweiten Ende 36 hängt von der Größe des Marknagels 1 ab, mit dem der Aufsatz 2 verbunden wird. Sie beträgt regelmäßig zwischen 43 und 54 mm bei einem Außendurchmesser des Aufsatzes von 11 und 15 mm. Der Durchmesser der verschiedenen Abschlussstücke 40 ist hierzu gleich und beträgt auch 11 bis 15 mm. Der Durchmesser der Durchbrechungen 31 in der Wand 30 des Aufsatzes 24 hängt davon ab, welche Befestigungsschrauben verwendet werden sollen und beträgt regelmäßig zwischen 3,5 und 4,5 mm. Alle genannten Teile bestehen aus einer Stahllegierung.

In Figur 9 ist der Aufsatz 24 aus den Figuren 4 bis 6 dargestellt, wie er mit der Abschlusskappe 6 des Marknagels 1 - und somit mit dem Marknagel 1 - aus Figur 1 verschraubt ist. Aus Gründen der Übersichtlichkeit ist der Rest des Marknagels 1 (siehe Figuren 1 bis 3) weggelassen.

In Figur 9 ist gut zu erkennen, wie die Festlegeschraube 45 in die Durchgangsöffnung 34 des Aufsatzes 24 und das Aufnahmegewinde 48 der Abschlusskappe 6 eingeschraubt ist und dadurch die Konusklemmung zwischen konischer Verbindungsfläche 29 am Aufsatz 24 und konischer Klemmfläche 46 an der Abschlusskappe 6 zu Stande kommt. Das Anziehen der Festlegeschraube 45 erfolgt durch die Öffnung 37 am zweiten Ende 36 des Befestigungsbereichs 26. Nachdem dies erfolgt ist und die Befestigungsschrauben durch die Durchbrechungen 31 in die in der Wand 30 gebildeten Führungskanäle 32, 38, 39 gesetzt wurden, wird die Öffnung 37 durch Einschrauben des Abschlussstücks 40 in das Verbindungsbereichinnengewinde 43 verschlossen.

Durch die Verbindung des Aufsatzes 24 mit dem Marknagel 1 kann auch eine Gelenkprothese am Marknagel - über den Aufsatz 24 - als eine Art Anker angebracht werden. Aufgrund der leichten Flexibilität der als Federn fungierenden Bänder 3, 4 wird eine leichte Bewegung des Knochens zugelassen, was eine schnellere Heilung erlaubt. Aufgrund des modularen Aufbaus von Marknagel 1 und Aufsatz 24 kann ohne die Notwendigkeit einer Explantation des Marknagels 1 der Gelenkteil einer Prothese getauscht werden, da dieser nur über den Aufsatz 24 mit dem Marknagel 1 verbunden ist; der Austausch erfolgt dann einfach dadurch, dass der Aufsatz 24 vom Marknagel 1 abgeschraubt wird und ein neuer Aufsatz 24, der an die benötigte Gelenkprothese angepasst ist, auf den Marknagel 1 aufgeschraubt wird.

### Bezugszeichenliste

- 1: Marknagel
- 2: Nagelkern
- 3: Äußeres Band
- 4: Inneres Band
- 5: Kopfstück
- 6: Abschlusskappe
- 7: Verriegelungsmechanismus
- 8: Außenbuchse
- 9: Innenbuchse
- 10: Außenkonus
- 11: Innenkonus
- 12: Außenzylinder
- 13: Innenzylinder
- 14: Endstück
- 15: Außengewinde
- 16: Nut
- 17: Erste Anschlagfläche
- 18: Zweite Anschlagfläche
- 19: Führungsteil
- 20: Innenfläche
- 21: Außenfläche
- 22: Schweißnaht
- 23: Hauptteil
- 24: Aufsatz
- 25: Verbindungsbereich
- 26: Befestigungsbereich
- 27: Abschlussstück
- 28: Aufnahmeausnehmung
- 29: Verbindungsfläche
- 30: Wand
- 31: Durchbrechung
- 32: Erster Führungskanal
- 33: Mittellängsachse
- 34: Durchgangsöffnung
- 35: Erstes Ende
- 36: Zweites Ende
- 37: Öffnung
- 38: Zweiter Führungskanal
- 39: Dritter Führungskanal
- 40: Abschlussstück
- 41: Gewindedurchbrechung
- 42: Schlitz
- 43: Verbindungsbereichinnengewinde
- 44: Abschlussstückaußengewinde
- 45: Festlegeschraube
- 46: Klemmfläche
- 47: Trennwand
- 48: Aufnahmegewinde

## Patentansprüche

1. Aufsatz (24) für das proximale Ende eines Marknagels (1)
mit einem Verbindungsbereich (25) an seinem ersten Ende (35) zum Verbinden des Aufsatzes (24) mit dem Marknagel (1)
und mit einem Befestigungsbereich (26) zur Verbindung mit mindestens einer Befestigungsschraube zur Festlegung des Aufsatzes (24) in einem Knochen,
wobei der Verbindungsbereich (25) ausgehend von seiner Abschlussfläche (27) eine sich in Richtung auf den Befestigungsbereich (26) erstreckende Aufnahmeausnehmung (28) aufweist, die zumindest in einem Teilbereich, ausgehend von der Abschlussfläche (27) eine sich verjüngende konische Verbindungsfläche (29) aufweist,
wobei der Befestigungsbereich (26) hohl ausgebildet ist und in seiner Wand (30) mindestens ein Paar einander zugeordneter Durchbrechungen (31) ausgebildet ist, wobei die Durchbrechungen (31) so gegenüber liegend in der Wand (30) angeordnet sind, dass sie einen ersten Führungskanal (32) zur Aufnahme der Befestigungsschraube bilden,
wobei zwischen Befestigungsbereich (26) und Verbindungsbereich (25) eine Trennwand (47) ausgebildet ist, in der eine Durchgangsöffnung (34) entlang der Mittellängsachse (33) vorhanden ist,
wobei am zweiten Ende (36) des Aufsatzes (24), das dem ersten Ende (35) abgewandt ist, eine Öffnung (37) vorhanden ist, die sich bis in den Befestigungsbereich (26) entlang der Mittellängsachse (33) erstreckt.

2. Aufsatz (24) nach Patentanspruch 1, wobei im Befestigungsbereich (26) ein zweiter Führungskanal (38) ausgebildet ist, dessen Durchbrechungen (31) so ausgerichtet sind, dass er durch die Mittellängsachse (33) verläuft, aber in Richtung der Mittellängsachse (33) gesehen nicht deckungsgleich zum ersten Führungskanal (32) ist.

3. Aufsatz (24) nach einem der Patentansprüche 1 oder 2, wobei im Befestigungsbereich (26) ein dritter Führungskanal (39) ausgebildet ist, dessen Durchbrechungen (31) so ausgerichtet sind, dass er in Richtung der Mittellängsachse (33) gesehen deckungsgleich zum ersten Führungskanal (32) ist, jedoch entlang der Mittellängsachse (33) beabstandet zum ersten Führungskanal (32) ist.

4. Aufsatz (32) nach einem der vorstehenden Patentansprüche, wobei in der Öffnung (37) am zweiten Ende (36) ein Abschlussstück (40) angeordnet ist, das entlang der Mittellängsachse (33) beabstandet zum ersten Führungskanal (32) ist.

5. Aufsatz (24) nach einem der vorstehenden Patentansprüche, wobei er mit einem Marknagel (1) verbunden ist, der in seinem Abschlussbereich eine konische Klemmfläche (46) aufweist, die im verbundenen Zustand spielfrei in der konischen Verbindungsfläche (29) des Verbindungsbereichs (25) angeordnet ist.

6. Aufsatz (24) nach Patentanspruch 5, wobei im Abschlussbereich des Marknagels (1) entlang der Mittellängsachse (33) ein Aufnahmegewinde (48) ausgebildet ist, das dieselbe Größe wie die Durchgangsöffnung (34) des Aufsatzes (24) hat, und wobei der Verbindungsbereich (25) des Aufsatzes (24) mittels einer Festlegeschraube (45) am Marknagel (1) festgelegt ist, die durch die Durchgangsöffnung (34) in das Aufnahmegewinde (48) eingedreht ist.

7. Aufsatz (24) nach einem der vorstehenden Patentansprüche, wobei der Marknagel (1) mit einem Nagelkern (2) und mit einem im vorderen Bereich des Marknagels (1) angeordneten Kopfstück (5) versehen ist, an dem der Anfang eines ersten Bandes (4) festgelegt ist, welches um den Nagelkern (2) herum aufgewickelt ist und das an dessen Ende im hinteren Bereich an einer Au βenbuchse (8) befestigt ist und welches bei einer Drehung entgegen seiner Aufwickelrichtung die radiale Kontur des Marknagels (1) erweitert, wobei der Nagelkern (2) in einer Innenbuchse (9) angeordnet ist und gegenüber dieser zwar rotierbar, aber nicht in axialer Richtung verschiebbar ist, wobei die Innenbuchse (9) innerhalb der Außenbuchse (8) rotierbar und in Längsrichtung des Marknagels (1) bewegbar ist, wobei zwischen der Innenbuchse (9) und der Außenbuchse (8) eine Konusfestlegevorrichtung ausgebildet ist, die in einer Verriegelungsposition keine Relativbewegung zwischen Innenbuchse (9) und Außenbuchse (8) zulässt und in einer axial dazu verschobenen Entriegelungsposition eine Rotation und eine Translation zwischen Innenbuchse (9) und Außenbuchse (8) zulässt.

8. Aufsatz (24) nach Patentanspruch 7, wobei die Konusfestlegevorichtung so ausgebildet ist, dass die Innenbuchse (9) einen Außenkonus (10) und die Außenbuchse (8) einen zum Außenkonus (10) koaxial ausgebildeten Innenkonus (11) aufweisen, wobei Außenkonus (10) und Innenkonus (11) sich jeweils in Richtung vom Kopfstück (5) weg verjüngen, sich gegenüber liegen und zumindest abschnittsweise gleich ausgebildet sind, wobei die Flächen von Außenkonus (10) und Innenkonus (11) in einer Verriegelungsposition aneinander gepresst sind und in Entriegelungspositionen kein Kraft- und Formschluss zwischen Außenkonus (10) und Innenkonus (11) gegeben ist.

9. Aufsatz (24) nach Patentanspruch 8, wobei beim Marknagel (1) die Innenbuchse (9) am kopfstückfernen Ende des Außenkonus (10) einen daran anschließenden Teil mit Außenzylinder (12) und die Außenbuchse (8) am kopfstückfernen Ende des Innenkonus (11) einen daran anschließenden Teil mit Innenzylinder (13) aufweisen, wobei Außenzylinder (12) und Innenzylinder (13) koaxial und im Wesentlichen spielfrei zueinander angeordnet sind.

10. Aufsatz (24) nach einem der Patentansprüche 8 oder 9, wobei am kopfstückfernen Ende des Nagelkerns (2) ein Endstück (14) mit einem Außengewinde (15) angeordnet ist, wobei zumindest ein Teil des Außengewindes (15) nicht von der Außenbuchse (8) in radialer Richtung überdeckt ist.

11. Aufsatz (24) nach einem der Patentansprüche 8 bis 10, wobei am kopfteilnahen Ende des Innenkonus (11) an der Außenbuchse (8) eine erste Anschlagfläche (17) und am kopfteilnahen Ende des Außenkonus (10) an der Innenbuchse (9) eine zweite Anschlagfläche (18) ausgebildet sind, die beide im Wesentlichen senkrecht zur Längsrichtung des Marknagels (1) orientiert sind.

12. Aufsatz (24) nach Patentanspruch 11, wobei die erste Anschlagfläche (17) Teil eines von der Außenbuchse (8) separaten Führungsteils (19) ist, wobei das Führungsteil (19) fest mit der Außenbuchse (8) verbunden ist.

13. Aufsatz (24) nach einem der Patentansprüche 8 bis 12, wobei der Nagelkern (2) gerade ausgebildet und mit der Innenbuchse (9) fest verbunden ist.

14. Aufsatz (24) nach einem der Patentansprüche 8 bis 13, wobei die Innenbuchse (9) und/oder der Nagelkern (2) hohl ausgebildet sind.

15. Aufsatz (24) nach einem der Patentansprüche 12 bis 14, wobei die Innenfläche (20) des Führungsteils (19) der Außenbuchse (8) zylindrisch ist und die darin angeordneten Teile der Außenfläche (21) der Innenbuchse (9) oder des Nagelkerns (2) im Wesentlichen dazu spielfrei ausgebildet sind.
